# EUROPÄISCHE PATENTSCHRIFT

(11) **EP 2 750 616 B1**
(45) Veröffentlichungstag und Bekanntmachung des Hinweises auf die Patenterteilung: **10.10.2018**
(21) Anmeldenummer: 12756684.2
(22) Anmeldetag: 30.08.2012
(51) Int. Cl.: A61B 17/74

(54) **VORRICHTUNG ZUM FIXIEREN EINES IM BEREICH DES OBERSCHENKELHALSES FRAKTURIERTEN KNOCHENS**
DEVICE FOR FIXING A BONE FRACTURED IN THE FEMORAL NECK REGION
DISPOSITIF D'IMMOBILISATION D'UN OS FRACTURÉ DANS LA ZONE DU COL DU FÉMUR

(30) Priorität: 02.09.2011 DE 102011112373; 20.10.2011 DE 102011116732
(43) Veröffentlichungstag der Anmeldung: 09.07.2014
(73) Patentinhaber: Königsee Implantate GmbH, 07426 Allendorf OT Aschau (DE)
(72) Erfinder: MAIER, Klaus-Jürgen, 83043 Bad Aibling (DE); FINGER, Ulrich, 07318 Saalfeld (DE); FAUCON, Mathilde, 92290 Châtenay-Malabry (FR); KRÜGER, Christian, 07422 Bad Blankenburg (DE)
(74) Vertreter: Kruspig, Volkmar
(86) Internationale Anmeldenummer: PCT/EP2012/066923
(87) Internationale Veröffentlichungsnummer: WO 2013/030312

(56) Entgegenhaltungen:
- JP-A- 11 299 804
- US-A1- 2005 182 405
- US-A1- 2008 082 101
- US-A1- 2009 143 825

## Beschreibung

Die Erfindung betrifft eine Vorrichtung zum Fixieren eines im Bereich des Oberschenkelhalses frakturierten Knochens, bestehend aus einer Gleithülsenplatte, die der Aufnahme eines Kraftträgers, insbesondere einer Schraube dient, und einer Trochanterabstützplatte, welche jeweils Ausnehmungen zur Aufnahme von Knochenschrauben aufweisen, wobei der Plattenkopf der Trochanterabstützplatte eine anatomisch angepasste Form besitzt und die Trochanterabstützplatte auf die Gleithülsenplatte aufsetz- und verschiebbar ausgeführt ist, gemäß Patentanspruch 1.

Aus der EP 0 347 874 A2 bzw. der US 4,988,350 ist eine Vorrichtung zum Verbinden eines im Bereich des Oberschenkelhalses gebrochenen Knochens vorbekannt. Diese vorbekannte Vorrichtung ist zweiteilig ausgebildet und besteht aus einer Knochenbefestigungslasche und einer Trochanterabstützplatte. Die Knochenbefestigungslasche weist eine in einem Winkel stehende Knochenschraubenaufnahmehülse auf, die unterhalb der Trochanterabstützfläche durch einen mit der Trochanterabstützplatte verbundenen, laschenförmigen Abschnitt hindurch geführt ist, der auf der Knochenbefestigungslasche aufliegt. Diese und der laschenförmige Abschnitt ist mit einer Anzahl von zur Deckung miteinander bringbaren Befestigungsschraubenaufnahmedurchbrechungen versehen, wobei die Trochanterabstützfläche eine der Trochanterform entsprechende Formgebung aufweist, so dass aufgrund der zweiteiligen Ausgestaltung eine verbesserte Anpassungsfähigkeit an anatomische Gegebenheiten durch beliebige Kombination der vorerwähnten Teile möglich ist. Nachteilig ist hier, dass die Trochanterplatte nicht nach Fixieren der Gleithülsenplatte anbringbar ist und keine Möglichkeit besteht, die Vorrichtung intraoperativ anatomisch anzupassen.

Die EP 0 515 828 B1 offenbart eine Trochanterstabilisierungsvorrichtung zur Versorgung von Schenkelhalsbrüchen mit zusätzlichen Trochanterfrakturen des Femur. Gemäß der dortigen Lösung soll eine nachträgliche Montage der Trochanterstabilisierungsplatte auf eine bereits eingebaute Hülsenlasche möglich werden. Um den festen Sitz der bereits montierten Hülsenlasche nicht wieder lockern zu müssen, ist eine Befestigungsperforation der Schaftpartie der Trochanterstabilisierungsplatte für das darin einzuführende Befestigungselement, vorzugsweise eine Knochenbefestigungsschraube, vollständig durchgängig ausgebildet.

Um die Trochanterstabilisierungsplatte gegen Verbiegung in der mediolateralen Ebene zu versteifen und um gleichzeitig eine Verdrehung der Trochanterstabilisierungsplatte gegenüber der Hülsenlasche zu verhindern, sind die Kanten der longitudinalen Schaftpartie mindestens in einem Teilbereich seitlich abgebogen, so dass sich eine Schiene ergibt, welche auf die am Knochen anliegende Knochenbefestigungslasche der Hülsenlasche aufgedrückt oder aufgeschnappt werden kann.

Bei einer weiteren Ausführungsform sind die longitudinale Schaftpartie der Trochanterstabilisierungsplatte und die Knochenbefestigungslasche der Hülsenlasche hohlzylindersektorförmig ausgebildet, um eine Anpassung an die Anatomie des annähernd kreiszylindrischen Röhrenknochens zu erreichen. Eine solche hohlzylindrische Ausgestaltung der Trochanterstabilisierungsplatte und der Knochenbefestigungslasche soll zudem die Biegesteifigkeit der Elemente verbessern. Die als äußere Schiene für die Knochenbefestigungslasche der Hülsenlasche ausgebildete Trochanterstabilisierungsplatte soll in Form einer elastischen Klammer gestaltet werden, welche nur mit den Enden ihrer Kanten die Knochenbefestigungslasche der Hülsenlasche federnd umschließt, so dass eine ansonsten angeblich kritische Passung der beiden ineinander greifenden Teile vermieden werden kann.

Bei der Trochanterstabilisierungsvorrichtung zur Fixation von Knochenfragmenten im Bereich des Hüftgelenks nach WO 2005/023127 A1 ist eine zentrale Platte mit mindestens einer Befestigungsperforation zur Aufnahme eines Knochenfixationsmittels vorhanden. Die Trochanterstabilisierungsplatte weist Arme auf, die es ermöglichen, Fragmente des großen Trochanters zusammenzuhalten und zu fixieren. Gewindelöcher im proximalen Teil der Trochanterstabilisierungsplatte erlauben eine stabile Fixierung von Fragmenten des großen Trochanters mit winkelstabilen Schrauben, d.h. Knochenschrauben, deren Schraubenkopf mittels eines Gewindes in der Trochanterstabilisierungsplatte verschraubt ist und sich daher gegenüber der Platte nicht verdrehen oder verschieben lässt. Weiterhin ist die vorbekannte Trochanterstabilisierungsplatte modular ausgestaltet, indem sie an die jeweilige Anatomie angeformt und zurechtgeschnitten werden kann. Bei einer Ausführungsform nach WO 2005/023127 A1 weist die zentrale Platte an ihren Längsseiten senkrecht zur Platte stehende Führungsschienen auf. Vorzugsweise entspricht die Breite der Knochenplatte dem Abstand der Führungsschienen, so dass die Knochenstabilisierungsmittel zusammen mit der zentralen Platte auf einer Knochenplatte parallel zu dieser verschiebbar sind.

Zusammenfassend sind Vorrichtungen zur Trochanterfixation bekannt, die Anwendungen in Kombination mit einer Vorrichtung zur extramedullären Osteosynthese von Schenkelhals- und pertrochantären Frakturen, insbesondere instabilen Frakturen unter Beteiligung des Trochanter major finden. Dabei wird von Gleithülsenplatten ausgegangen. Diese bestehen aus einer Knochenplatte mit Schraubenlöchern in Längsrichtung zur Verschraubung auf der lateralen Kortikalis des Femur und mit einer integrierten Gleithülse, die mit der Platte einen Winkel bildet, sowie zur Aufnahme eines durch den Schenkelhals in den Femurkopf eingebrachten Kraftträgersystems geeignet ist. Durch die mögliche Kombination von Platten unterschiedlicher Länge mit unterschiedlichen Winkeln ergibt sich ein nutzbares System von Gleithülsenplatten für verschiedene individuelle anatomische Gegebenheiten und Fraktursituationen. Allerdings reicht bei instabilen Frakturen unter Beteilung des Trochanter major die Gleithülsenplatte mit Kraftträgersystem durch den Schenkelhals in den Femurkopf nicht aus. Hier muss eine zusätzliche Vorrichtung zur Trochanterfixation in Kombination mit der Gleithülsenplatte angewendet werden.

JP 11 299804 A offenbart eine Vorrichtung gemäß dem Oberbegriff von Anspruch 1.

Ausgehend vom Stand der Technik ist es daher Aufgabe der Erfindung, eine weiterentwickelte Vorrichtung zum Fixieren eines im Bereich des Oberschenkelhalses frakturierten Knochens anzugeben, bestehend aus einer Gleithülsenplatte und einer Trochanterabstützplatte, welche jeweils Ausnehmungen zur Aufnahme von Knochenschrauben aufweisen, und wobei der Plattenkopf der Trochanterabstützplatte eine anatomisch angepasste oder anpassbare Form besitzt. Die Trochanterabstützplatte soll auf die Gleithülsenplatte aufsetz- und verschiebbar ausgeführt sein, so dass sich eine laterale Abstützung in optimaler Weise und gleichzeitig eine hohe mechanische Stabilität zur Aufnahme zyklischer Belastungen einstellt. Die erfindungsgemäße Vorrichtung soll entweder gemeinsam mit der Gleithülsenplatte oder aber bei bereits liegender Gleithülsenplatte bedarfsweise einsetzbar sein. Gegenstand der Erfindung ist darüber hinaus eine Schablone zur intraoperativen Bestimmung der jeweiligen Halslänge der einzusetzenden Trochanterabstützplatte.

Die Lösung der Aufgabe der Erfindung erfolgt durch eine Vorrichtung gemäß der Merkmalskombination nach Patentanspruch 1, wobei die Unteransprüche mindestens zweckmäßige Ausgestaltungen und Weiterbildungen beinhalten.

Es wird demnach von einer Vorrichtung zum Fixieren eines im Bereich des Oberschenkelhalses frakturierten Knochens ausgegangen, diese bestehend aus einer Gleithülsenplatte und einer Trochanterabstützplatte, welche jeweils Ausnehmungen zur Aufnahme von Knochenschrauben aufweisen, wobei der Plattenkopf der Trochanterabstützplatte eine anatomisch angepasste Form besitzt und die Trochanterabstützplatte auf die Gleithülsenplatte aufsetz- und verschiebbar ausgeführt ist.

Erfindungsgemäß wird die Trochanterabstützplatte quasi wie ein Schuh auf das Schaftende der Gleithülsenplatte aufgeschoben und kann dann gemeinsam mit der Gleithülsenplatte am Knochen fixiert werden. Eine konstruktive Änderung an der Gleithülsenplatte mit möglicherweise notwendiger Materialschwächung ist nicht erforderlich.

Die schuhartige Profilführungs-Steckverbindung weist eine sehr hohe Stabilität auf, insbesondere können große Abstützkräfte aufgenommen werden.

Durch das gemeinsame Verschrauben des Schafts auf der Plattenoberseite mit der Gleithülsenplatte auf dem Knochen wird die Trochanterabstützplatte in axialer Richtung fixiert und kann sich nicht unbeabsichtigt verschieben.

Es ist also erfindungsgemäß mindestens am zum Plattenkopf weisenden Ende die Trochanterabstützplatte mit einer die Gleithülsenplatte hintergreifenden Profilführung ausgeführt derart, dass der hierzu komplementäre Abschnitt der Gleithülsenplatte schuhartig aufgenommen ist.

Die hintergreifende Profilführung geht vom plattenkopfnahen zum plattenkopffernen Ende in eine offene, die Gleithülsenplatte lediglich seitlich stützende Führung über.

Bei einer Ausführungsform der Erfindung ist die Führung als formgepaarte Führung, insbesondere als Flachführung oder prismatische Führung ausgebildet.

Bei einer weiteren Ausführungsform der Erfindung geht die Profilführung plattenkopfseitig in eine taschenförmige Ausnehmung über, um das hierzu komplementäre Ende der Gleithülsenplatte allseitig stabilisierend zu umgreifen und zu fixieren.

Ausgestaltend besitzt die Trochanterabstützplatte im Bereich zwischen Plattenkopf und hintergreifender Profilführung eine Sollbiegestelle.

Die Sollbiegestelle ist bevorzugt im Bereich zwischen Plattenkopf und Plattenschaft ausgebildet.

Die Sollbiegestelle kann durch eine Materialschwächung des Plattenmaterials in einfacher Weise realisiert werden.

Die Plattenlöcher im jeweiligen Plattenschaft sind als Langlöcher ausbildbar, wobei der Plattenkopf eine anatomisch optimierte Löffelform aufweist.

Am Plattenkopf ist erfindungsgemäß ein Zielblock aufsetz- und befestigbar.

Die Trochanterabstützplatte ist mit Gleithülsenplatten kombinierbar, welche eine unterschiedliche Länge und/oder einen unterschiedlichen Winkel zwischen Plattenlängsachse und Gleithülse aufweisen.

Am Plattenkopf der Trochanterabstützplatte sind Ausnehmungen und/oder Bohrungen für chirurgische Fäden bzw. Kirschnerdraht vorgesehen.

Diese Ausnehmungen und/oder Bohrungen können unterschnitten ausgeführt werden.

Die erfindungsgemäße Schablone zur Halslängenbestimmung der Trochanterabstützplatte besteht aus einer Schablonengleithülsenplatte, einer Schablonentrochanterabstützplatte mit einem auf ihrer Oberseite aufgebrachten Maßstab, wobei die Schablonentrochanterabstützplatte unterseitig eine Führung zum gleitbeweglichen Verschieben relativ zur Schablonengleithülsenplatte aufweist. Weiterhin sind Mittel zum Fixieren der jeweiligen Verschiebeposition vorhanden.

Die Schablonentrochanterabstützplatte weist einen Plattenkopf und einen Plattenschaft, ähnlich der operativ einzusetzenden Trochanterabstützplatte auf. Der Maßstab der Schablonentrochanterabstützplatte ist auf der Oberseite des Plattenschafts gut sichtbar aufgebracht und es besitzt der Plattenschaft eine in Schaftlängsrichtung orientierte Langlochausnehmung.

Die Schablonengleithülsenplatte weist bevorzugt eine verkürzte Führungshülse auf und es ist im Plattenbereich eine Gewindebohrung eingebracht, welche zur Aufnahme einer Schablonenfixierschraube dient.

Der Kopf der Schablonenfixierschraube kommt im Einsatzzustand der Schablone an der Oberseite der Schablonentrochanterabstützplatte zur Anlage, wobei der Gewindeabschnitt der Schraube die Langlochausnehmung durchdringt. Durch Festdrehen der Schraube kann die jeweilige Lageposition bzw. Verschiebeposition zwischen der Schablonengleithülsenplatte und der Schablonentrochanterabstützplatte fixiert werden.

Es liegt im Sinne der Erfindung, die Schablone so auszubilden, dass ein Satz von Schablonengleithülsenplatten mit unterschiedlichen CCD-Winkeln bereitgehalten wird.

Hinweise zur Korrektur abgelesener Längen auf dem Maßstab der Schablonentrochanterabstützplatte können dort entsprechend angegeben und dargestellt werden.

Die Handhabung der Schablone zur Halslängenbestimmung der dann operativ einzusetzenden Trochanterabstützplatte stellt sich wie folgt dar.

Zunächst wird die Schablonentrochanterabstützplatte auf die Schablonengleithülsenplatte aufgeschoben und dort mit Hilfe der Schablonenfixierschraube arretiert. In diesem vormontierten Zustand wird dann die gebildete Schablonenanordnung in die eingebrachte Bohrung auf den Knochen aufgesetzt. Konkret nimmt hierbei die Bohrung im Knochen die bevorzugt verkürzte Führungshülse der Schablonengleithülsenplatte auf.

Im Anschluss wird die Schablonenfixierschraube gelockert und die Schablonentrochanterabstützplatte bis zum Inanschlagkommen des Plattenkopfes am Trochanter vorgeschoben. Hiernach erfolgt ein Anziehen der Schablonenfixierschraube. Am Maßstab kann nunmehr bezüglich eines vorgebbaren Fixpunkts die Halslänge der zu verwendenden Trochanterabstützplatte ermittelt bzw. abgelesen werden.

Es sei an dieser Stelle angemerkt, dass die Schablonenanordnung grundsätzlich auch ohne Einsatz der Schablonenfixierschraube funktionsfähig ist, jedoch verbessert letztere die Handhabung der Gesamtanordnung und vermeidet Messfehler.

Die Erfindung soll nachstehend anhand eines Ausführungsbeispiels sowie unter Zuhilfenahme von Figuren näher erläutert werden.

Hierbei zeigen:
- Fig. 1: eine Unterseitenansicht der erfindungsgemäßen Vorrichtung;
- Fig. 2: eine Seitenansicht der erfindungsgemäßen Vorrichtung mit Bohrhülse und aufgesetztem Zielblock;
- Fig. 3: eine Draufsicht auf die Vorrichtung gemäß Fig. 2;
- Fig. 4: eine Seitenansicht der Schablonengleithülsenplatte;
- Fig. 5: eine Draufsicht der Schablonengleithülsenplatte;
- Fig. 6: Seitenansicht und Ansicht der Oberseite der Schablonentrochanterabstützplatte mit erkennbarem Maßstab zum Ablesen des entsprechenden Messwerts;
- Fig. 7: Seitenansicht und Draufsicht der Schablonengleithülsenplatte mit aufgesetzter Schablonentrochanterabstützplatte;
- Fig. 8: eine Darstellung der bevorzugt zum Einsatz kommenden Schablonenfixierschraube mit Vielkantausnehmung im Schraubenkopf und Schraubengewindeabschnitt;
- Fig. 9: Seitenansicht und Draufsicht einer Darstellung ähnlich derjenigen nach Fig. 7, jedoch mit eingebrachter Schablonenfixierschraube;
- Fig. 10: eine Prinzipdarstellung der Schablonenanordnung in Draufsicht und Seitenansicht, wobei die Seitenansicht den angedeuteten Knochen umfasst und sich die Führungshülse der Schablonengleithülsenplatte in einer bereits eingebrachten Bohrung im Knochen befindet, und
- Fig. 11: eine Darstellung ähnlich derjenigen nach Fig. 10, jedoch mit verschobener Schablonentrochanterabstützplatte in Richtung des Trochanters, und zwar in einer solchen Position, dass sich der Plattenkopf der Schablonentrochanterabstützplatte in Anlage mit dem Trochanter befindet.

Für dieselben Funktionselemente und Baugruppen in den Figuren werden identische Bezugszeichen verwendet.

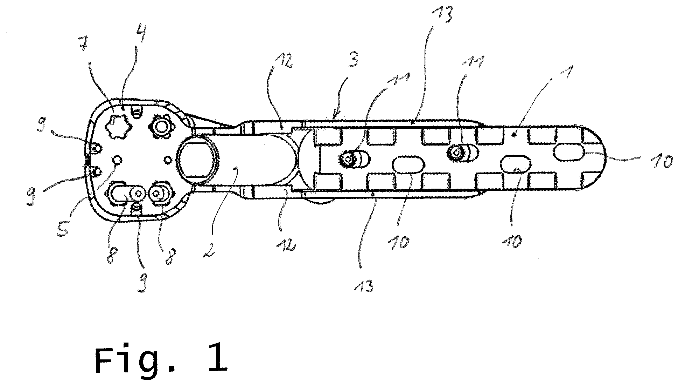

Gemäß Fig. 1 wird zunächst die Unterseite der Gleithülsenplatte 1 ersichtlich, und zwar nebst Führungshülse 2.

Zwischen der Längsachse der Gleithülsenplatte 1 und der Führungshülse 2 besteht ein vorgegebener sogenannter CCD-Winkel z.B. in einem Bereich zwischen 115° und 150°.

Die Gleithülsenplatte 1 ist mit einer Trochanterabstützplatte 3 kombinierbar, welche einen Plattenkopf 4 aufweist, vorzugsweise ausgebildet in Löffelform.

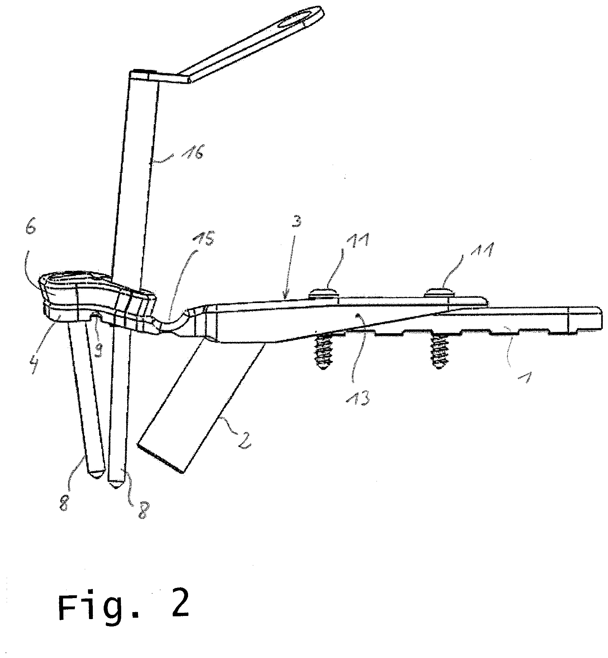

Im Plattenkopf 4 ist eine Gewindebohrung 5 für die Befestigung des Zielblocks 6 (siehe Fig. 2) vorhanden. Weiterhin sind verschiedene Schraubenlöcher 7 zur Befestigung von Knochenschrauben 8 ausgebildet. Die Schraubenlöcher 7 sind bei der gezeigten Ausführungsform so realisiert, dass eine winkelvariabelstabile Fixierung möglich wird. Weitere Ausnehmungen 9 dienen zur Fadenfixierung bzw. zum Fixieren eines Kirschnerdrahts.

Langlöcher 10 in der Gleithülsenplatte 1 sowie entsprechend komplementär in der Trochanterabstützplatte 3 dienen ebenfalls der Aufnahme von Knochenschrauben 11.

Am zum Plattenkopf 4 weisenden Ende besitzt die Trochanterabstützplatte 3 eine die Gleithülsenplatte 1 hintergreifende Profilführung 12. Diese Profilführung 12 ist als formgepaarte Führung, insbesondere Flachführung, so ausgeführt, dass der diesbezüglich komplementäre Abschnitt der Gleithülsenplatte 1 schuhartig umgreifend aufgenommen wird.

Die hintergreifende Profilführung 12 geht vom plattenkopfnahen zum plattenkopffernen Ende in eine offene, die Gleithülsenplatte 1 lediglich seitlich stützende Führung bzw. einen diesbezüglichen Führungsabschnitt 13 über.

Dieser seitliche Führungsabschnitt 13 kann einen stetig oder unstetig ansteigenden Verlauf besitzen, wie dies aus der Fig. 2 ersichtlich ist.

Im Bereich zwischen Plattenkopf 4 und der hintergreifenden Profilführung 12 besitzt die Trochanterabstützplatte 3 eine Sollbiegestelle 15 (siehe Fig. 2), die z.B. in Form einer dezidierten Materialschwächung ausbildbar ist.

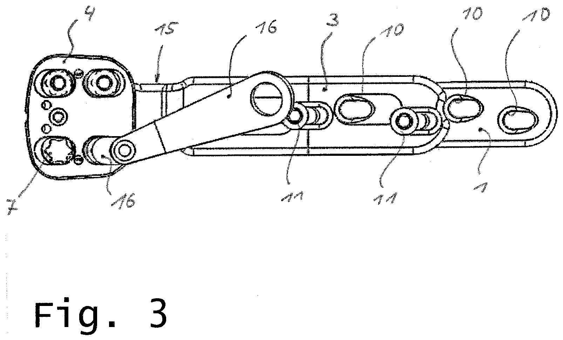

Die Fig. 2 und 3 lassen eine aufgesetzte Bohrhülse 16 und die Fig. 2 den bereits erwähnten Zielblock 6 erkennen.

Die in der Fig. 3 von ihrer Oberseite gezeigten Langlöcher 10 können auch als spezielle Kompressionslöcher ausgeführt werden.

Die schuhartige Profilführung / Steckverbindung weist eine sehr hohe Stabilität auf, so dass sich eine insgesamt rigide Verbindung der Platten einstellt. Wie bereits kurz erläutert, können am proximalen und den lateralen Rändern des Plattenkopfes wahlweise zusätzliche Fadenfixations- bzw. Kirschnerdrahtlöcher angeordnet sein. Durch Unterschneidung der Platte ausgehend von den Löchern zum Plattenrand hin können die Löcher auch bei liegender Platte auf dem Knochen mit einer gebogenen chirurgischen Nadel passiert werden.

Die Löffelform des Plattenkopfes sichert eine optimale Abstützung. Um dennoch individuellen Besonderheiten und unterschiedlichen Winkeln der Hülsenplatte Rechnung zu tragen, kann der Abstand zwischen Plattenkopf und Schaft in verschiedenen Längen ausgeführt werden. Die erwähnte Sollbiegestelle 15 ermöglicht das intraoperative Biegen der Platte, ohne die Schraubenlöcher zu verformen, so dass eine individuelle Anpassung an die Knochenmorphologie gegeben ist.

Die Sollbiegestelle ist durch eine definierte Schwächung des Querschnitts der Verbindung von Plattenkopf und Plattenschaft an der Ober- und/oder an der Unterseite realisierbar. Die Schraubenlöcher 7 im Plattenkopf 4 haben eine Geometrie, die sowohl eine einfache winkelstabile Verschraubung ermöglicht als auch eine variabel winkelstabile Verschraubung. Die winkelstabil eingebrachten Schrauben 8 dienen der Fixierung von Knochenfragmenten und der Abstützung des Kopf-Hals-Fragments.

Durch Fixierung des Zielblocks 6 am Plattenkopf 4 auf der Plattenoberseite wird bei variabel winkelstabiler Verschraubung der Winkel hinsichtlich des absoluten Wertes und der zulässigen Richtung gezielt und definiert eingeschränkt.

Die erfindungsgemäße Vorrichtung ermöglicht unter Beseitigung der Nachteile des bekannten Standes der Technik eine laterale Abstützung durch anatomische Formung und Ausführung in verschiedenen, kombinierbaren Längen. Gleichzeitig wird eine hohe mechanische Stabilität zur Aufnahme von zyklischen Belastungen sowie eine stabile Verankerung mit der Gleithülsenplatte erreicht, wobei die Vorrichtung entweder gemeinsam mit der Gleithülsenplatte oder bei bereits liegender Gleithülsenplatte bei Bedarf angebracht werden kann. Schraubenlöcher im proximalen Bereich zur Aufnahme variabel winkelstabiler Schrauben dienen der Fixierung von Trochanterfragmenten und zur Abstützung des Kopf-Hals-Fragments. Weiterhin sind Fadenfixations- bzw. Kirschnerdrahtlöcher am proximalen und/oder an lateralen Rändern in Schrägstellung oder Unterscheidungsausbildung vorhanden und es ist wenigstens eine Befestigungsbohrung für einen Zielblock vorgesehen, wobei letzterer zur Begrenzung des Schwenkbereichs beim Einbringen von winkelvariabel fixierbaren Schrauben dient.

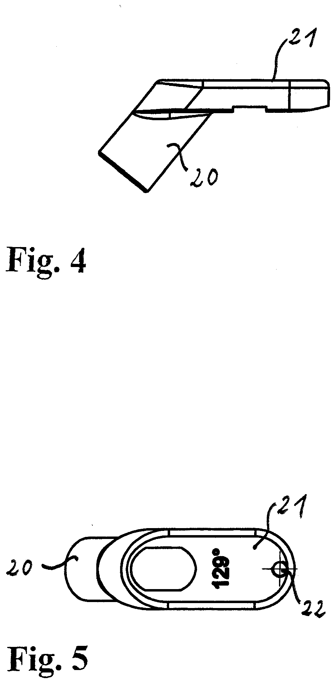

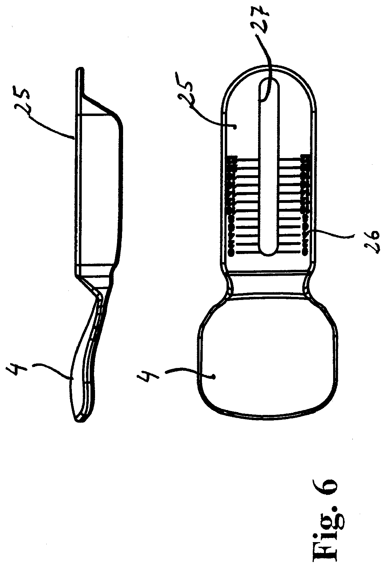

Die erfindungsgemäße Schablone zur Halslängenbestimmung der operativ einzusetzenden Trochanterabstützplatte soll unter Zuhilfenahme der Fig. 4 bis 11 nachstehend erläutert werden.

Die Schablonenanordnung zur Halslängenbestimmung umfasst zunächst eine in den Fig. 4 und 5 in Seitenansicht und Draufsicht dargestellte Schablonengleithülsenplatte.

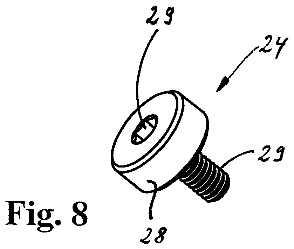

Die Schablonengleithülsenplatte besitzt bei einer Ausführungsform eine verkürzte Führungshülse 20 und einen Plattenbereich 21. Am in Fig. 5 gezeigten hinteren Ende des Plattenbereichs 21 ist eine Gewindebohrung 22 zur Aufnahme einer Schablonenfixierschraube 24 (siehe Fig. 8) gezeigt.

Die Schablonentrochanterabstützplatte der Schablonenanordnung entspricht der prinzipiellen Ausbildung der eigentlichen Trochanterabstützplatte, d.h. die Schablonentrochanterabstützplatte weist einen Plattenkopf 4 sowie einen Plattenschaft 25 auf.

Der Plattenkopf 4 ist hierbei anatomisch geformt.

Auf der Oberseite des Plattenschafts 25 ist eine Skala bzw. ein Maßstab 26 zum Ablesen eines Wertes zur Halslängenbestimmung der Trochanterabstützplatte aufgebracht.

Darüber hinaus befindet sich im Plattenschaft eine Langlochausnehmung 27.

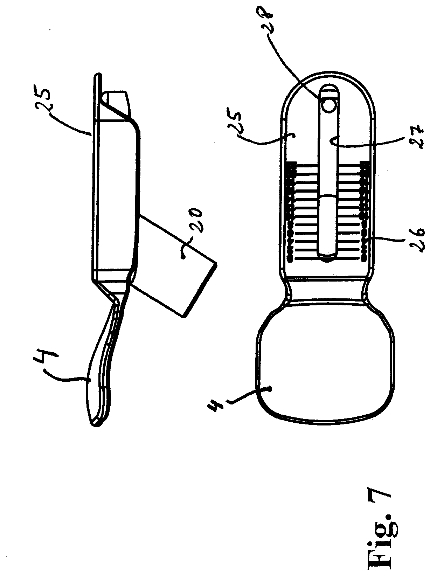

Gemäß der Seitenansicht sowie der Draufsicht nach Fig. 7 wird die Schablonengleithülsenplatte von der Schablonentrochanterabstützplatte über eine an der Schablonentrochanterabstützplatte unterseitig vorgesehene Führung gleitbeweglich aufgenommen.

Die Schablonenfixierschraube 24 weist einen Schraubenkopf 28 sowie einen Gewindeabschnitt 29 auf. Im Schraubenkopf 28 ist ein Innenvielkant 29 zum Ansetzen eines entsprechenden Werkzeugs vorhanden.

Mit Hilfe der Schablonenfixierschraube 24 kann die entsprechende Verschiebeposition zwischen Schablonengleithülsenplatte und Schablonentrochanterabstützplatte fixiert werden.

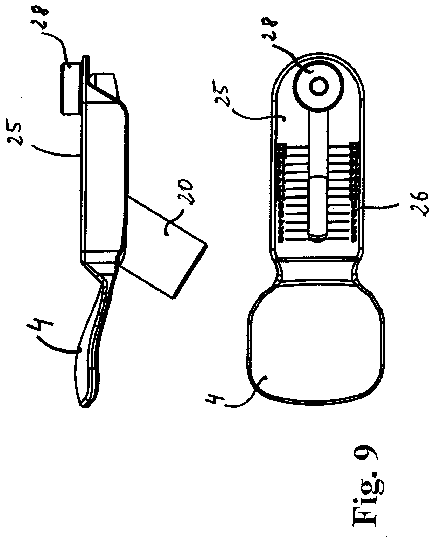

Die komplette Schablonenanordnung, umfassend Schablonengleithülsenplatte, Schablonentrochanterabstützplatte und Schablonenfixierschraube ist in der Seitenansicht sowie in der Draufsicht nach Fig. 9 gezeigt

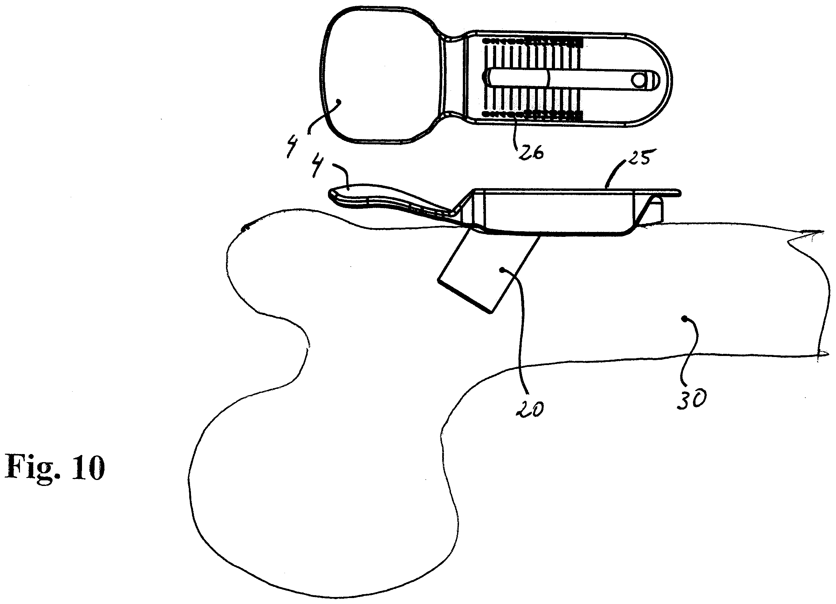

Im entsprechend montierten Zustand wird die Schablonenanordnung gemäß Fig. 10 in eine Bohrung des Knochens 30 eingesetzt. In diesem Zustand befindet sich die Führungshülse 20 in der vorerwähnten Bohrung des Knochens 30.

Diesbezüglich sei auf die Fig. 10 aufmerksam gemacht.

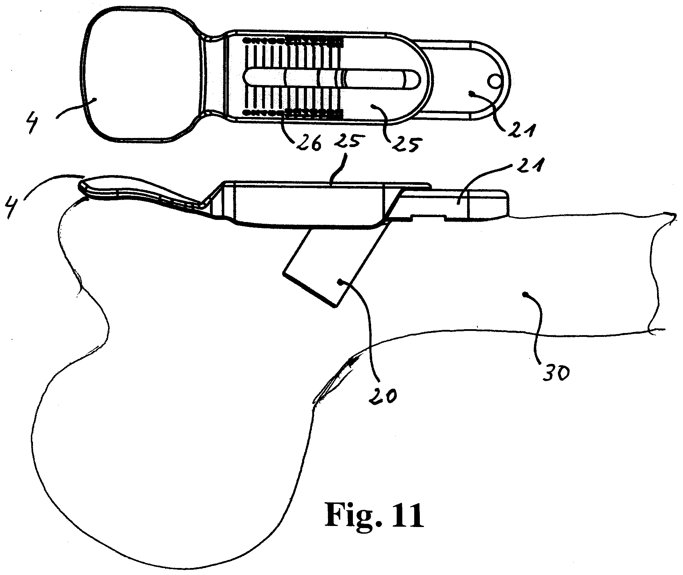

Gemäß Darstellung nach Fig. 11 wird dann die Schablonentrochanterabstützplatte mit ihrem Plattenkopf 4 so lange bewegt, bis ein entsprechender Anschlag am Trochanter gegeben ist. Hiernach kann dann die entsprechende Halslänge der operativ zu nutzenden Trochanterabstützplatte mit Hilfe des Maßstabs 26 abgelesen werden.

## Patentansprüche

1. Vorrichtung zum Fixieren eines im Bereich des Oberschenkelhalses frakturierten Knochens, bestehend aus einer Gleithülsenplatte (1) und einer Trochanterabstützplatte (3) mit Plattenkopf (4), welche jeweils Ausnehmungen (7) zur Aufnahme von Knochenschrauben (8) aufweisen, wobei der Plattenkopf (4) der Trochanterabstützplatte (3) eine anatomisch angepasste Form besitzt und die Trochanterabstützplatte (3) auf die Gleithülsenplatte (1) aufsetz- und verschiebbar ausgeführt ist, wobei mindestens am zum Plattenkopf (4) weisenden Ende die Trochanterabstützplatte (3) eine die Gleithülsenplatte (1) hintergreifende formgepaarte Profilführung (12) aufweist derart, dass der hierzu komplementäre Abschnitt der Gleithülsenplatte (1) schuhartig aufgenommen ist, **dadurch gekennzeichnet, dass** die hintergreifende Profilführung (12) vom plattenkopfnahen zum plattenkopffernen Ende in eine offene, die Gleithülsenplatte (1) lediglich seitlich stützende Führung (13) übergeht.

2. Vorrichtung nach Anspruch 1, **dadurch gekennzeichnet, dass**
die formgepaarte Führung als Flachführung ausgebildet ist.

3. Vorrichtung nach einem der vorangegangenen Ansprüche,
**dadurch gekennzeichnet, dass**
in der Gleithülsenplatte (1) und in der Trochanterabstützplatte (3) Langlöcher (10) ausgebildet sind.

4. Vorrichtung nach einem der vorangegangenen Ansprüche,
**dadurch gekennzeichnet, dass**
der Plattenkopf (4) eine Löffelform aufweist, ein oder mehrere Bohrungen (7) zur Aufnahme von Knochenschrauben und/oder Gewindebohrungen (5) aufweist.

5. Vorrichtung nach einem der vorangegangenen Ansprüche,
**dadurch gekennzeichnet, dass**
am Plattenkopf (4) eine Gewindebohrung (5) für die Befestigung eines Zielblocks (6) ausgebildet ist.

6. Vorrichtung nach einem der vorangegangenen Ansprüche,
**dadurch gekennzeichnet, dass**
am Plattenkopf (4) der Trochanterabstützplatte (3) Ausnehmungen (9) und/oder Bohrungen für Fäden oder Kirschnerdraht vorgesehen sind.

7. Vorrichtung nach Anspruch 6,
**dadurch gekennzeichnet, dass**
die Ausnehmungen (9) und/oder Bohrung unterschnitten sind.

8. Vorrichtung nach einem der vorangegangenen Ansprüche,
**dadurch gekennzeichnet, dass**
die Profilführung (12) plattenkopfseitig in eine taschenförmige Ausnehmung übergeht, um das hierzu komplementäre Ende der Gleithülsenplatte (1) allseitig stabilisierend zu fixieren.

9. Vorrichtung nach einem der vorangegangenen Ansprüche,
**dadurch gekennzeichnet, dass**
die Trochanterabstützplatte im Bereich zwischen Plattenkopf und hintergreifender Profilführung eine Sollbiegestelle aufweist.

10. Schablonenanordnung zur Halslängenbestimmung der Trochanterabstützplatte (3) gemäß Vorrichtung nach einem oder mehreren der vorangegangenen Ansprüche, bestehend aus einer Schablonengleithülsenplatte, einer Schablonentrochanterabstützplatte mit einem auf ihrer Oberseite aufgebrachten Maßstab (26), wobei die Schablonentrochanterabstützplatte unterseitig eine Führung zum gleitbeweglichen Verschieben relativ zur Schablonengleithülsenplatte aufweist, und Mittel zum Fixieren (24) der jeweiligen Verschiebeposition.

11. Schablone nach Anspruch 10,
**dadurch gekennzeichnet, dass**
die Schablonentrochanterabstützplatte einen Plattenkopf (4) und einen Plattenschaft (25) aufweist, wobei der Maßstab (26) auf der Oberseite des Plattenschafts (25) aufgebracht ist und der Plattenschaft (25) eine in Schaftlängsrichtung orientierte Langlochausnehmung (27) besitzt.

12. Schablonenanordnung nach Anspruch 10 oder 11,
**dadurch gekennzeichnet, dass**
die Schablonengleithülsenplatte eine verkürzte Führungshülse (20) aufweist und im Plattenbereich (21) eine Gewindebohrung (22) eingebracht ist, welche zur Aufnahme einer Schablonenfixierschraube (24) dient.

13. Schablonenanordnung nach Anspruch 12,
**dadurch gekennzeichnet, dass**
der Kopf (28) der Schablonenfixierschraube (24) an der Oberseite der Schablonentrochanterabstützplatte zur Anlage kommt und der Gewindeabschnitt (29) der Schablonenfixierschraube (24) die Langlochausnehmung (27) durchdringt.

## Claims

1. Device for fixing a bone fractured in the region of the femoral neck, consisting of a sliding sleeve plate (1) and a trochanter support plate (3) with plate head (4) each comprising recesses (7) for receiving bone screws (8), the plate head (4) of the trochanter support plate (3) having an anatomically adapted shape and the trochanter support plate (3) being embodied in such a way that it can be displaceably mounted on the sliding sleeve plate (1), wherein
at least at the end facing the plate head (4), the trochanter support plate (3) comprises a form-paired profile guide (12) which engages behind the sliding sleeve plate (1) in such a way that the portion of the sliding sleeve plate (1) which is complementary thereto is received in a shoe-like manner, **characterized in that**
the engaging profile guide (12) changes from the end near the plate head to the end far from the plate head into an open guide (13) supporting the sliding sleeve plate (1) only laterally.

2. Device according to Claim 1, **characterized in that** the form-paired guide is designed as a flat guide.

3. Device according to one of the preceding claims, **characterized in that** slotted holes (10) are formed in the sliding sleeve plate (1) and in the trochanter support plate (3).

4. Device according to one of the preceding claims, **characterized in that** the plate head (4) has a spoon shape and comprises one or more bores (7) for receiving bone screws and/or threaded bores (5).

5. Device according to one of the preceding claims, **characterized in that** a threaded bore (5) for fastening an aiming block (6) is formed at the plate head (4).

6. Device according to one of the preceding claims, **characterized in that** recesses (9) and/or bores for threads or Kirschner wire are provided at the plate head (4) of the trochanter support plate (3)

7. Device according to Claim 6, **characterized in that** the recesses and/or bore are undercut.

8. Device according to one of the preceding claims, **characterized in that** the profile guide (12) on the plate head side turns into a pocket-shaped recess in order to fix the complementary end of the sliding sleeve plate (1) on all sides in a stabilizing manner.

9. Device according to one of the preceding claims, **characterized in that** the trochanter support plate comprises a predetermined bending point in the region between the plate head and the engaging profile guide.

10. Template arrangement for determining the neck length of the trochanter support plate (3) pursuant to the device according to one or more of the preceding claims, consisting of a template sliding sleeve plate, a template trochanter support plate having a scale (26) placed on its upper side, the template trochanter support plate having a guide on the bottom side for sliding displacement relative to the template sliding sleeve plate, and fixing means (24) for the respective displacement position.

11. Template according to Claim 10, **characterized in that** the template trochanter support plate comprises a plate head (4) and a plate shaft (25), wherein the scale (26) is placed on the upper side of the plate shaft (25) and the plate shaft (25) having a slotted hole recess (27) oriented in the longitudinal direction of the shaft.

12. Template arrangement according to Claim 10 or 11, **characterized in that** the template sliding sleeve plate comprises a shortened guide sleeve (20) and a threaded bore (22) is formed in the plate region, the threaded bore serving to receive a template fixing screw (24).

13. Template arrangement according to Claim 12, **characterized in that** the head (28) of the template fixing screw (24) rests on the upper surface of the template trochanter support plate and the threaded portion (29) of the template fixing screw (24) penetrates the slotted hole recess (27).

## Revendications

1. Dispositif de fixation d'un os fracturé dans la zone du col du fémur, constitué par une plaque à douille coulissante (1) et par une plaque d'appui de trochanter (3) pourvue d'une tête de plaque (4), qui présentent chacune des évidements (7) pour loger des vis à os (8),
dans lequel
la tête de plaque (4) de la plaque d'appui de trochanter (3) possède une forme adaptée sur le plan anatomique et la plaque d'appui de trochanter (3) est réalisée de manière à pouvoir être posée et déplacée en translation sur la plaque à douille coulissante (1),
au moins à l'extrémité dirigée vers la tête de plaque (4), la plaque d'appui de trochanter (3) comprend un guidage profilé (12) de forme complémentaire engageant par l'arrière la plaque à douille coulissante (1), de telle sorte que la portion complémentaire de la plaque à douille coulissante (1) est logée à la manière d'une chaussure,
**caractérisé en ce que**
le guidage profilé (12) d'engagement par l'arrière se transforme en un guidage ouvert (13) soutenant seulement latéralement la plaque à douille coulissante (1) depuis l'extrémité proche de la tête de plaque vers l'extrémité éloignée de la tête de plaque.

2. Dispositif selon la revendication 1,
**caractérisé en ce que**
le guidage de forme complémentaire est réalisé sous forme de guidage plat.

3. Dispositif selon l'une des revendications précédentes,
**caractérisé en ce que**
des trous oblongs (10) sont ménagés dans la plaque à douille coulissante (1) et dans la plaque d'appui de trochanter (3).

4. Dispositif selon l'une des revendications précédentes,
**caractérisé en ce que**
la tête de plaque (4) présente une forme de cuillère, un ou plusieurs perçages (7) pour loger des vis à os et/ou des perçages taraudés (5).

5. Dispositif selon l'une des revendications précédentes,
**caractérisé en ce que**
un perçage taraudé (5) est ménagé au niveau de la tête de plaque (4) pour fixer un bloc cible (6).

6. Dispositif selon l'une des revendications précédentes,
**caractérisé en ce que**
des évidements (9) et/ou des perçages pour des fils ou pour une broche de Kirschner sont prévus au niveau de la tête de plaque (4) de la plaque d'appui de trochanter (3).

7. Dispositif selon la revendication 6,
**caractérisé en ce que**
les évidements (9) et/ou les perçages sont en contre-dépouille.

8. Dispositif selon l'une des revendications précédentes,
**caractérisé en ce que**
du côté tête de plaque, le guidage profilé (12) se transforme en un évidement en forme de poche pour fixer l'extrémité complémentaire de la plaque à douille coulissante (1) en la stabilisant de tous les côtés.

9. Dispositif selon l'une des revendications précédentes,
**caractérisé en ce que**
la plaque d'appui de trochanter présente un emplacement destiné au coudage dans la zone entre la tête de plaque et le guidage profilé d'engagement par l'arrière.

10. Ensemble de gabarit pour déterminer la longueur de col de la plaque d'appui de trochanter (3) selon le dispositif selon l'une ou plusieurs des revendications précédentes, constitué par une plaque à douille coulissante de gabarit, par une plaque d'appui de trochanter de gabarit pourvue d'une échelle (26) appliquée sur sa face supérieure, la plaque d'appui de trochanter de gabarit présentant du côté inférieur un guidage pour le déplacement coulissant par rapport à la plaque à douille coulissante de gabarit, et des moyens (24) de fixation dans la position de coulissement respective.

11. Gabarit selon la revendication 10,
**caractérisé en ce que**
la plaque d'appui de trochanter de gabarit présente une tête de plaque (4) et une tige de plaque (25), l'échelle (26) étant appliquée sur la face supérieure de la tige de plaque (25) et la tige de plaque (25) possédant un évidement en trou oblong (27) orienté en direction longitudinale de la tige.

12. Ensemble de gabarit selon la revendication 10 ou 11,
**caractérisé en ce que**
la plaque à douille coulissante de gabarit présente une douille de guidage raccourcie (20), et un perçage taraudé (22) est ménagé dans la zone de plaque (21) qui sert à loger une vis de fixation de gabarit (24).

13. Ensemble de gabarit selon la revendication 12,
**caractérisé en ce que**
la tête (28) de la vis de fixation de gabarit (24) vient en appui sur la face supérieure de la plaque d'appui de trochanter de gabarit et la portion taraudée (29) de la vis de fixation de gabarit (24) traverse l'évidement en trou oblong (27).
